(19) Europäisches Patentamt / European Patent Office / Office européen des brevets

(11) **EP 4 618 094 A1**

(12) **EUROPEAN PATENT APPLICATION**

(43) Date of publication:
**17.09.2025 Bulletin 2025/38**

(21) Application number: **24163216.5**

(22) Date of filing: **13.03.2024**

(51) International Patent Classification (IPC):
**G16H 20/40** (2018.01)

(52) Cooperative Patent Classification (CPC):
**G16H 20/40**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Nipro Corporation**
**Settsu-shi, Osaka 566-8510 (JP)**

(72) Inventor: **VASTA, Alessandro**
**Osaka, 566-8510 (JP)**

(74) Representative: **Meissner Bolte Partnerschaft mbB**
**Patentanwälte Rechtsanwälte**
**Postfach 86 06 24**
**81633 München (DE)**

(54) **METHOD FOR PROCESSING, PREFERABLY PERIODICAL, MEASUREMENT SIGNALS TO BE DISPLAYED ON A USER DEVICE**

(57) Method for processing, preferably periodical, measurement signals, in particular from a measurement device for blood pressure, to be displayed on a user device comprising at least one visual user interface, the method comprising the following steps:
a) receiving a measurement signal from at least one sensor at a first time;
b) associating the measurement signal with a first display-region of a plurality of display-regions of a display;
c) calculating, based

- on the measurement signal,
- on the first time and
- on a previous display-state-parameter of at least one of the plurality of display regions at a second time,
at least one display-state-parameter of the at least one of the plurality of display-regions,
d) assigning a new display-state-parameter to at least one of the plurality of display-regions, based on the display-state-parameter calculated according to c).

Fig.1

**Description**

Many modern diagnostic methods and medical procedures rely on an accurate and ongoing monitoring of certain key parameters, which may directly or indirectly be linked to a patient's medical condition.

In many situations it is important to accurately assess both the current state of a patient and a general trend in the measurements over a certain amount of time. In particular, it is important to make the relevant aspects of the measurement history readily available to a user. At the same time however, the commonly utilized user interfaces, such as displays for example, may vary extensively in their format and capabilities, such as memory, resolution, brightness etc. This requires a compromise between the extent to which the full history of the measurements is displayed and the degree of compression with which the information is "summarized".

Hence, there exists the general problem of displaying the measurement outcomes in a way that allows both for a quick identification of the current state of the measurement and the general trend over a, preferably adjustable, window in the past, in a manner that is viable with a wide variety of displays.

In particular, the problem is solved by a method for processing, preferably periodical, measurement signals, in particular from a measurement device for blood pressure, to be displayed on a user device comprising at least one visual user interface, the method comprising the following steps:

a) receiving a measurement signal from at least one sensor at a first time;
b) associating the measurement signal with a first display-region of a plurality of display-regions of a display;
c) calculating, based

-   on the measurement signal,
-   on the first time and
-   on a previous display-state-parameter of at least one of the plurality of display regions at a second time,

at least one display-state-parameter of the at least one of the plurality of display-regions,
d) assigning a new display-state-parameter to at least one of the plurality of display-regions, based on the display-state-parameter calculated according to c).

A key aspect of the invention is the calculation of the display state parameter based on both a measurement signal and a previous display state parameter. By combining information about the most recent measurement as well as information about previous display state parameter, it is possible to efficiently illustrate both the current physical state, being measured, and a record of the past measurement history. In particular, this allows an efficient detection of patterns and trends within the data, which manifest themselves in a drift of the measurement results/signals. This is particularly useful, because many indications are more readily identified, when considering the entire history of measurements of a given parameter, instead of focusing merely on snapshots. At the same time, this allows for a compact representation of the measurement data, without providing the full details in the form of individual measurement results, thereby not overburdening the user.

According to this aspect of the invention, the display state parameter of a given display region is influenced both by the current measurement signal at the first time and the previous state existing at a second time, wherein the second time predated the first time. Here, the display state parameter being calculated based on the measurement signal encompasses the possibility that an adjustment to the measurement signal is made based on the absence of a measurement signal.

This bears the additional advantage of saving both memory and display space, as the historical information about a certain measurement signal remains available, at least to some extent, without saving the entire history explicitly. Hence, sufficient information about the detailed history of the measurement signal may be provided in a compact form even on small displays.

A display-state-parameter refers to any parameter, which sets the state of a display region, while the display is in use. For instance, the display state parameter may be a parameter that sets a certain brightness and/or color and/or color intensity and/or the like in a specific region of the display.

A display region can in general be any connected subset of the area of the display in question. For instance, the display may preferably be subdivided in a checkerboard pattern, wherein every square corresponds to a display region. In particular, it is preferable that the state of the display within a given display region is set entirely by associated display state parameter. Thus, there may be a one to one correspondence between the display regions and the displayed state parameters. Alternatively, more than one display state parameter may be associated with the given display region. For example, a given display region may have associated with it both a display state parameter for the brightness and another display state parameter for the color.

According to a preferred embodiment, each of the display regions is indicative of a range of at least one measurement parameter.

Thus, the measurement signals, specifically certain measurement values, are associated with specific display regions. For example, it may be preferable to associate a specific region of the display with a measurement, which falls into a certain predetermined range (bin) of the parameter being measured. This establishes a one-to-one correspondence between the different display regions and measurement outcomes.

This greatly facilitates the fast and accurate portrayal of the measurement information on the visual user interface, in particular allowing for the user to easily read-off the desired information. Furthermore, the correspondence between parameter ranges and display regions may be used to straightforwardly process measurement input data into display data.

Display data, in particular, comprises the information about the location of the display regions on the visual user interface as well as the plurality of display-state-parameters associated with the corresponding regions.

In general it is possible to simplify such an assignment, e.g. if a significant part of the measurement signal (information) is provided by its integer part. If this is not the case, the measurement signal may be brought to an integer value by a function. Examples of such a function may be an int()-function.

In a further preferred embodiment, the method comprises an initialization step of predefining a plurality of display regions, in particular based on a region of at least one measurement parameter, wherein the plurality of display regions preferably covers the entire range of the at least one measurement parameter.

Here, a region of the parameter refers, in particular, to the entire range of parameter values, which may reasonably be expected to be a possible measurement outcome. As blood pressures for example will typically vary between 0 and 250 mmHg, the region of the blood pressure parameter may, for example be defined as 0 to 275 mmHg, or more preferably 0 to 300 mmHg.

As the display size and format may vary depending on the specific hardware available, this step further improves the readability and resolution of the displayed data. In particular, it allows for the adaptation of the data processing to both the expected measurement region and the physical dimensions of the display.

In a preferred embodiment, the number of display regions may therefore be chosen such as to give an appropriate resolution over the entire or a part of the parameter region, with the given display size. In particular, it can be ensured that the number of display regions is sufficiently low, to ensure that the physical size of the display region on the display is large enough to be discriminated.

According to another preferred embodiment, the resolution can vary over the region of the parameter. For example, while the entire region may cover values from 0 to 300, it may be the case that most measurements are expected only in a range between 100 and 200. In this case, a greater number of display regions (or bins) may be associated with this smaller range, in order to maximize the resolution in the areas most critical to the specific parameter under scrutiny. This allows to condense large parameter regions appropriately, and still provide meaningful display data for small displays.

According to a further preferred embodiment, the calculation of the at least one display-state-parameter in step c) comprises adding a predetermined contribution-parameter to the at least one display-state-parameter, when the measurement signal was found to correspond to the associated display-region.

The predetermined contribution-parameter may be predetermined in the sense that the same, in particular constant, parameter value is added every time a measurement signal was found to correspond for the associated display-region. The contribution parameter can but must not be constant in time and can in particular be a function of the display region as well, as long as the amount by which is increased or decreased is known in advance (predetermined).

For instance, in a preferred embodiment at least one of a color, a brightness or a color intensity of a display-region is increased or decreased by a predetermined amount, every time a measurement associated with said display region is registered.

This bears the advantage that display regions, which correspond to measurements, which were repeatedly registered, can easily be distinguished from those that have not yet occurred. Furthermore, this provides a direct correlation between the state of the display region and the frequency with which the corresponding measurement outcome occurred.

In a further preferred embodiment the calculation of the at least one display-state-parameter in step c) comprises the application of a damping-factor to the at least one display-state-parameter, wherein the damping factor depends on a retaining time and the first and/or second time, wherein the damping factor preferably does not depend on a recorded measurement signal.

This allows to set a general trend with which the display state parameters change as measurement iterations increase. In particular, this allows to accumulate or damp the effects of measurements on the state of the display over time.

A preferred implementation of the damping factor is given by the multiplication of the display state parameter by the following forgetting factor:

$$forgetting\ factor = fading^{\mathrm{Ts/Tr}}$$

Here, the factor *fading* is a residual percentage of the display state parameter. For instance, the factor may preferably be the residual color intensity percentage after a number of input samples of a potential cell at the initial maximum color intensity, supposing no more input occurred to increment that particular cell value.

Tr refers to the retaining time, wherein the retaining time is a measure of the general time scale for which a measurement outcome will continue to effect the display state parameter. Preferably, the retaining time is the time to guarantee a maximum residual color intensity (= fading* sat) if no more input occurred to increment that particular display region.

Sat is the saturation, a value codifying the maximum display state parameter intensity

Ts is the sampling time of the input of the measurement updating the display state.

However, other forgetting factors are possible, such as for example an exponential behavior and/or a linear behavior and/or a logarithmic behavior (etc.).

In a further preferred embodiment the predetermined contribution-parameter and/or damping factor is chosen such that the display-state-parameter is bounded from above and/or below.

This ensures that the calculated parameters meet the physical requirements of the display itself. For instance, if the display is only adapted to display the parameter up to a certain intensity (for example maximum brightness or maximum color saturation or the like) the bound can be chosen appropriately to reflect this. Furthermore, in preferred embodiments forgetting factor should be bounded between 0 and 1, as values below 0 would result in an unintentional sign, while values of greater than 1 (while possible and in some cases preferable) do not result in an actual damping.

It is a general feature that the display state parameters, fading, sat, and Tr are configurable according to the user needs and the display graphical rendering. As a general placeholder for the display state parameter $\alpha$ will be used throughout the application. As explained above, in particular we will have $0 < \alpha < sat$ and $0 < fading < 1$.

In a further preferred embodiment, the damping factor is chosen such that display-state-parameters tend to decrease over time, wherein the decrease can preferably be adjusted to depend on the retaining time according to one of the following: constant, linear, polynomial, exponential, logarithmic.

This preferred feature enables to establish that certain measurements are "forgotten" over time. As the state of the display is meant to reflect the state of a physical object, such as the patient, as concisely as possible, this forgetting effect bears several advantages. Firstly, the most relevant information for the user is displayed at a greater intensity, as the most recent measurements are the ones least effected by damping. Secondly, as all measurement sensors are prone to deliver output data which is subject to some degree of error, the forgetting of older results enables to eliminate outliers, as they will have a low chance of being repeated, while being subject to constant damping.

According to a further preferred embodiment, the method comprises a further step of automatically choosing a predetermined display-state-parameter for the first display-region.

While the state of the display regions give an indication of the history of the measurements for the measurement parameter range to which the region responds, it is oftentimes necessary to identify the most recent measurement. As such, it is preferred to automatically set the display state parameter of the first display region to a certain value. In particular, it is preferential to choose a display state parameter, which could not otherwise be obtained by the time-dependent updates, such as the application of the forgetting factor. For example, in a case in which the display state parameter related to the forgetting factor as refers to a color intensity, it may be chosen to set the display color to a different color for the first region, while maintaining intensity. After a new measurement corresponding to a different first region, this color change may be reversed, such that the display region corresponding to the most recent measurement always has a different color from the remaining display state regions.

In a further preferred embodiment, the method further comprises a step of calibrating the user device such that every pair of distinct display-state-parameters leads to a visually distinguishable pair of display-states.

As the resolution, brightness, color fidelity and other factors may vary both from display to display and in particular for a given display over time, this step may be used to ensure that the display state parameters, which are calculated and assigned based on method describe above, remain distinguishable to the user. This is especially helpful, as it allows for the use of a wider variety of displays and for easier maintenance as displays show signs of aging.

In a further preferred embodiment the plurality of display-regions correspond to intervals of a blood pressure.

The use of the method is particularly well suited for the display of blood pressures, as monitoring of blood pressures, preferably venous blood pressures, requires the tracking of trends in the blood pressure over time, while simultaneously placing an emphasis on more recent blood pressures over previous ones. However, the method may also be useful for other applications in which a (specific) window of a history of the measurement is of relevance, such as energy consumption.

In a further preferred embodiment a plurality of display-state-parameters are defined, in particular stored, as a state vector, wherein each of the components of the state vector is associated to a display-region and/or a measurement parameter.

This greatly facilitates the processing of the display state parameters, as the vector format provides an easy form of component-wise manipulation in the form of standard vector calculus. In particular, this is advantageous from a hardware perspective, as reading, saving and retrieving of the necessary entries can be implemented efficiently on the hardware, while the vector-component structure provides a natural ordering for the association with the display-state-regions.

According to a preferred embodiment, the display-state-parameters represent at least one of the following: a display-brightness, a display-color, a color intensity.

The display-brightness, a display-color, and/or color intensity are particularly suited to be supported by the widest variety of displays, while at the same time being the most easily distinguishable for the eye of a human user, such as a nurse or doctor.

Here, preferably, color intensity refers to the shade of a particular color chosen. For example, intensity may be varied from a very dark (intense) blue to a very light (non-intense) shade of blue. Further preferably, this may be achieved either by applying a grey factor between 0 and 1 to a given base color (such as blue), or by adjusting the transparency of a given base color between 0 and 1.

In a further preferred embodiment, the damping factor is applied by the multiplication of every vector component with the damping factor.

This is particularly useful as vector multiplication is very efficient and provides an overall damping factor for the entirety of the display state parameters.

Another aspect of the invention is given by a system comprising at least one sensor and a user device comprising at least one visual user interface, preferably a measurement device for blood pressures, as well as a processor controlling the visual user interface based on a measurement signal from the sensor, wherein the system is configured to carry out a method according to the description above.

With a system according to this aspect, the same advantages, which were discussed previously in relation to the method, can be achieved.

In a further preferred embodiment, the invention includes a Haemodialysis or blood circuit machine comprising a system according to the description above.

According to another aspect, the invention includes a computer readable medium storing instructions that when executed by at least one processor cause the at least one processor to implement a method according to the description above.

In the following the preferred embodiments will be described in greater detail with respect to the Figures. The figures show:

Fig 1. A view of the display state after a first measurement.

Fig 2. A view of the display state after a second measurement.

Fig 3. A view of the display state after a plurality of measurements.

Fig. 4A A measurement graph of a venous blood pressure over time.

Fig. 4B A display state resulting from the pressure graph displayed in figure 4A.

Fig. 5A A measurement graph of a venous blood pressure over time.

Fig. 5B A display state resulting from the pressure graph displayed in figure 5A.

Fig. 6A A measurement graph of a venous blood pressure over time.

Fig. 6B A display state resulting from the pressure graph displayed in figure 6A.

In a particular embodiment of the present invention the display state parameter may be given by the color intensity of the display region and the measurement signal corresponds to a venous blood pressure.

In this case, the display 1 may essentially be split up in order to constitute a bar graph, wherein the horizontal axis corresponds to the measurement parameter.

The bar graph appearance of the (for example blue) faded horizontal bar representing the past trend of the acquired variable can be initialized on a vector hereinafter called "bar_graph_vect" with as many components (cells) as the possible different distinct values of blood pressure that should be displayed in a bar graph.

If the minimum value expected for the blood pressure measurement outcomes is VP0 and the maximum value is VPn, the vector size will be:

$$N = int(VPn) - int(VP0) + 1;$$

where int() is a function extracting the integer part (rounded) of the respective value.

Let us suppose that a significant part of the information VP is provided by its integer part. If this is not the case, a proper scaling factor should be integrated in the int()-function to make integer the desired significant part of the information.

For illustration: "New" denotes the current index of the vector "bar_graph_vect" and can be calculated as: New = int(VP) - int(VPO) +1.

At the acquisition start all components (cells) of the vector "bar_graph_vect" may be initialized to a value of 0.

So, every time a new value VP is acquired (corresponding to a new measurement), the following calculation may be performed to obtain the color intensity of each bar graph position, which may correspond to a display region 2, 3, 4, 5, that is represented by each cell content of the vector "bar_graph_vect":

- New = int(VP) - int(VPO) +1; This selects the correct vector entry, corresponding to the appropriate display region 2, 3, 4, 5.

- bar_graph_vect[New] = bar_graph_vect[New] + $\alpha$; This adjusts the display-state-parameter of the associated display region 2, 3, 4, 5 by adding a predetermined contribution parameter, based on the fact that a measurement was registered in the corresponding interval.

- bar_graph_vect = bar_graph_vect*_forgetting_factor;_ This adjusts the color intensity of all cells, based on the discrepancy between the number of sample times lying between the last measurement of the corresponding range and the present (first) time.

As a further step it may be implemented that the display state parameter does not exceed an upper bound (in this case the saturation), even when the corresponding measurement range is measured often successively:
If(bar_graph_vect[New] > _sat_) bar_graph_vect [New] = _sat_

In this embodiment $\alpha$ is the graph vector additional color intensity contribution. It may be a constant or a function of the value in bar_graph_vect[New] with a value always < _sat_. (In the following example in Table 1 $\alpha$ is constant with $\alpha$=0.05).

$\alpha$ may be a fraction of the maximum color intensity (_sat_). Furthermore,

$$forgetting\ factor = fading^{\frac{1}{K}};$$

where _forgetting factor_ is always < 1 by construction.

As _fading_ may be the residual color intensity percentage (<1) after K input samples of a potential cell at the initial maximum color intensity (sat), supposing no more VP input occurred to increment that particular cell value, the above holds true.

Here K = Tr/Ts. The retaining time may be the time to guarantee a maximum residual color intensity (_fading*sat_) if no more VP input occurred to increment that particular cell value, and Ts is the sampling time of the input function VP updating the bar graph. The sampling time updating the current VP measure may be different (preferably more frequent) than Ts.

Here: _sat_ is a constant (the value codifying the maximum color intensity).

All parameters $\alpha$, _fading, sat_ and _Tr_ are configurable according to the user needs and the display graphical rendering. (0 < $\alpha$ < sat and 0 < _fading_ < 1)

The following table shows a numerical example where VP is simulated with a normal distribution values sequence with average 150 and standard deviation $\sigma$ = 3. The visualization parameters are: $\alpha$ = 0.05, fading = 10% (0.1), sat = 1, VP is sampled with Ts = 5sec and the retaining time is
Tr = 1800 sec = 30 min (K = 360) leading to a forgetting_factor = 0.9936.

The table shows the portion of the cells surrounding the average expected value for some values of the time t.

| New <- int(VP)-int(VP0) +1 | | 144 | 145 | 146 | 147 | 148 | 149 | 150 | 151 | 152 | 153 | 154 | 155 | 156 |
|---|---|---|---|---|---|---|---|---|---|---|---|---|---|---|
| Bar_graph vect content in the | t=0 | 0.00 | 0.00 | 0.00 | 0.00 | 0.00 | 0.00 | 0.00 | 0.00 | 0.00 | 0.00 | 0.00 | 0.00 | 0.00 |
| | t=1*Ts | 0.00 | 0.00 | 0.00 | 0.00 | 0.00 | 0.05 | 0.00 | 0.00 | 0.00 | 0.00 | 0.00 | 0.00 | 0.00 |
| middle segment with index New in [144:156] | t=2*Ts | 0.00 | 0.00 | 0.00 | 0.00 | 0.00 | 0.05 | 0.05 | 0.00 | 0.00 | 0.00 | 0.00 | 0.00 | 0.00 |
| | t=5*Ts | 0.000 | 0.000 | 0.000 | 0.049 | 0.000 | 0.098 | 0.049 | 0.000 | 0.000 | 0.050 | 0.000 | 0.000 | 0.000 |
| | t=10*Ts | 0.000 | 0.000 | 0.097 | 0.097 | 0.000 | 0.095 | 0.047 | 0.050 | 0.000 | 0.097 | 0.000 | 0.000 | 0.000 |
| | t=50*Ts | 0.041 | 0.040 | 0.199 | 0.162 | 0.089 | 0.246 | 0.479 | 0.262 | 0.223 | 0.164 | 0.091 | 0.088 | 0.000 |
| | t=100*Ts | 0.068 | 0.114 | 0.236 | 0.372 | 0.281 | 0.442 | **0.694** | 0.446 | 0.360 | 0.243 | 0.112 | 0.105 | 0.089 |
| | t>retaining_t | 0.156 | 0.336 | 0.387 | **0.693** | **0.815** | **0.950** | **0.924** | **0.994** | **0.717** | **0.689** | 0.363 | 0.193 | 0.088 |

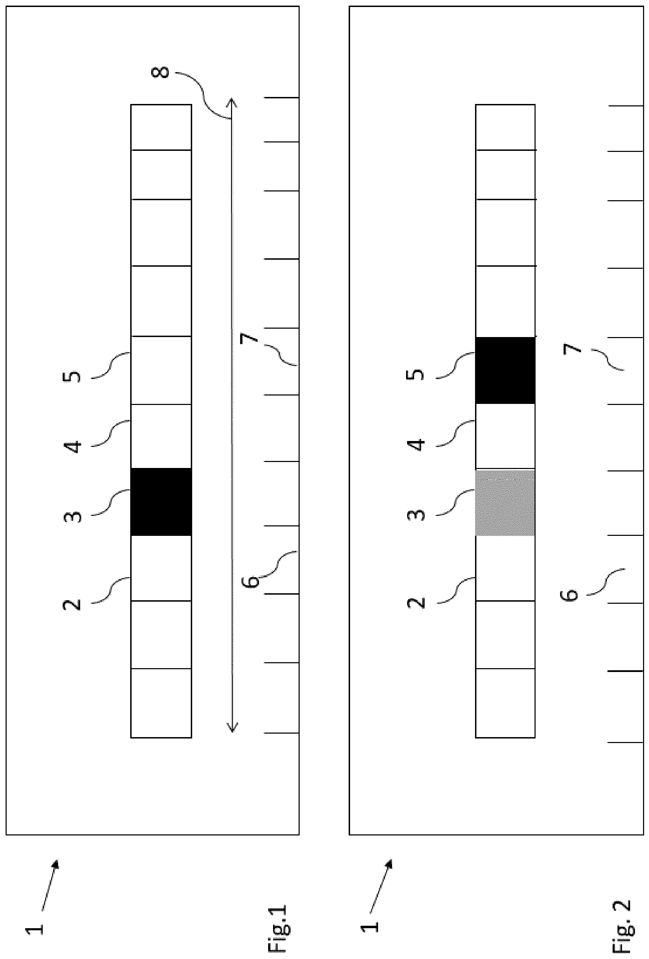

Figure 1 illustrates the inventive concept in a more abstract setting. Again, the display state parameter was chosen to be a color intensity. The display 1 is split up into a plurality of display regions 2, 3, 4, 5, which are each associated with a specific range 6, 7 etc. of the measurement parameter, which are shown here on the bottom of the display 1 for illustration.

In the shown example, the region of the measurement parameter 8 spans the plurality of all measurement parameter ranges, as indicated.

As this depict the situation after the first measurement, there is only one display region 3 which corresponds to a measurement outcome. Accordingly, the display state parameter of the said region is updated according to the procedure(s) described above. Here, an embodiment is shown where the first display region 3, is automatically set to have a display specified display state parameter, in this case setting color saturation to the maximum (saturation) value. In the embodiment shown, all other display regions 2, 3, 4, 5 where initialized with a valueof the display state parameter of 0.

Figure 2 shows the same display 1 at a point in time where a second measurement has been performed. In this example the most recently measured (first) display region 5 is set to the automatic value of color intensity. Then, as the forgetting factor is applied to all, or preferably all other, display regions 2, 3, 4, the color intensity of the display region 3 is decreased to a value differing from the saturation value.

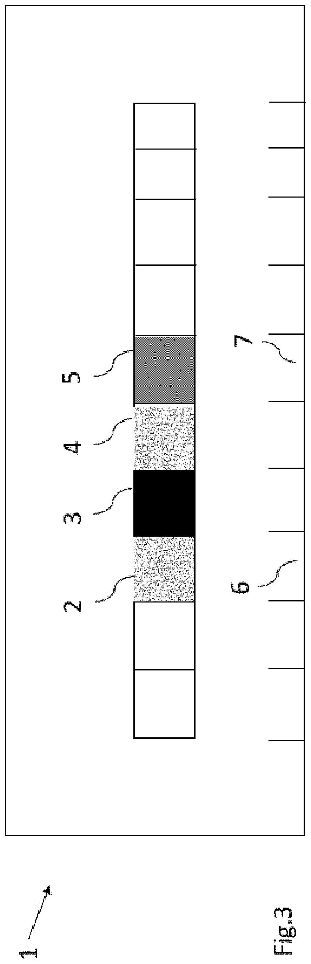

Figure 3 shows the situation after a couple of iterations. Here, the display region 3 is once more the first region corresponding to the measurement signal. In this case the remaining display regions 2, 4 and 5 have been updated to reflect the measurement history of the patient. In particular, it can be seen that the measurement range 6 corresponding to the display region 2 has been measured less frequently and/or less recently than the measurement range 7 corresponding to the display region 5.

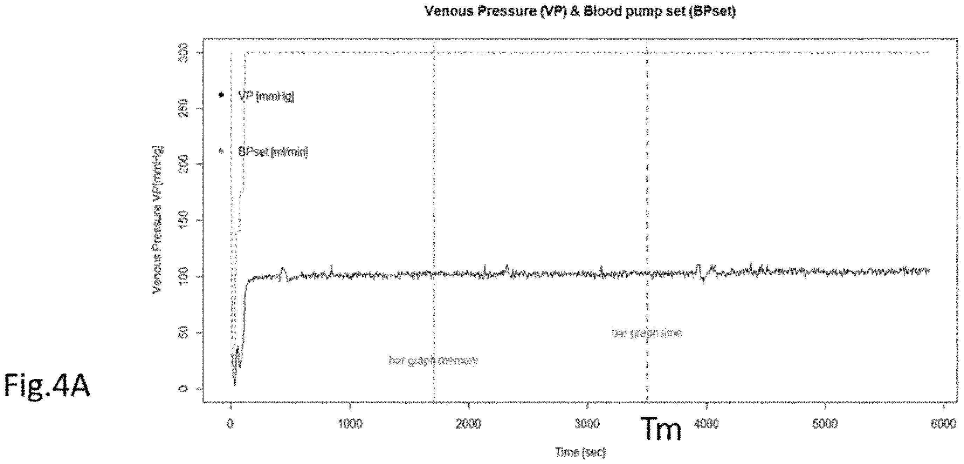

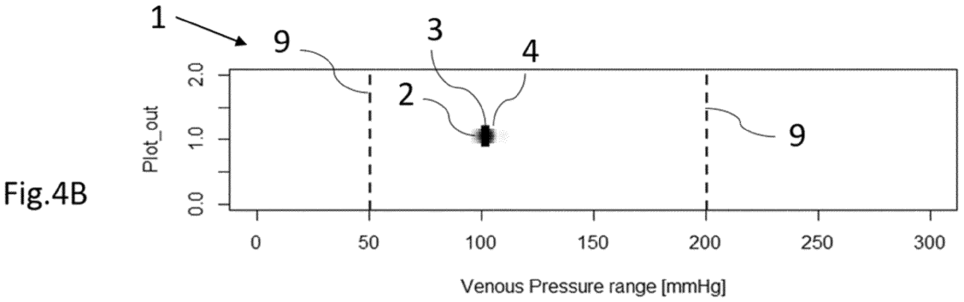

Figure 4A shows a more realistic depiction of the measurement graph of a measurement outcome for venous pressure over time. The rightmost dashed vertical line indicates the time at which the view of the display 1 shown in figure 4B is shown. It thus forms the measurement time for figure 4B. The vertical line labelled bar graph memory, shows the earliest time, for which the contribution of a measurement to the display state parameters has not fully decayed yet. The distance between this line and Tm will of course be a function of the retaining time, wherein larger retaining times lead to larger distances (i.e. a longer memory).

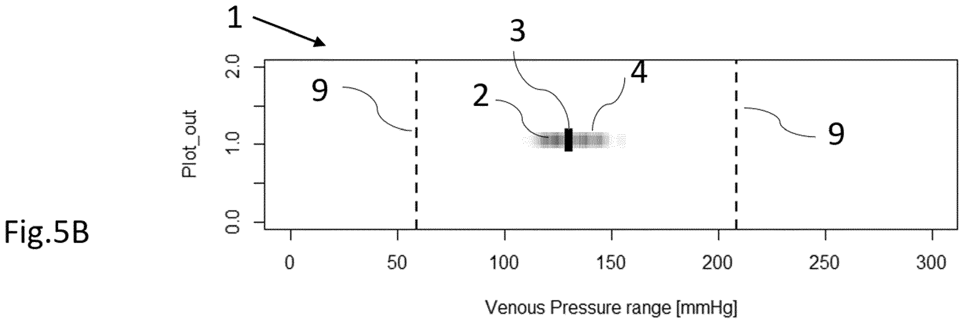

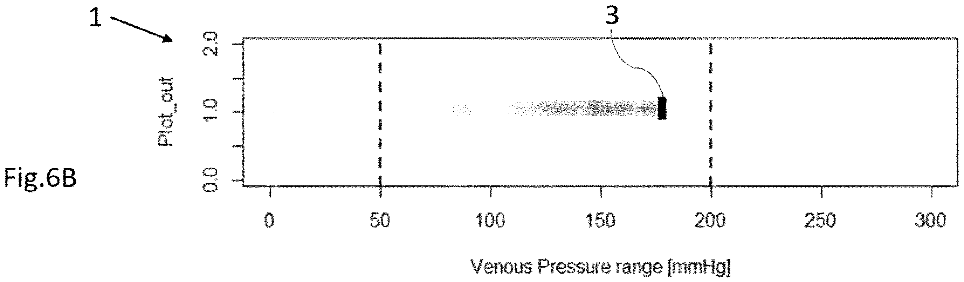

Figure 4B depicts the state of the display 1 resulting from the graph shown in figure 4A at time tm. As can be seen, due to the relatively constant blood pressure, the display regions 2 and 4 are relatively equal in display state parameter. Here display region 3 corresponds to the first display region. The alarm thresholds 9 are thresholds for which an alarm may be triggered if a predetermined number of measurements exceed this threshold. In Figure 4B (and the following figure 5B and 6B), a respective pixel may correspond with a respective display region 2, 3, 4 and 5 in Figure 1.

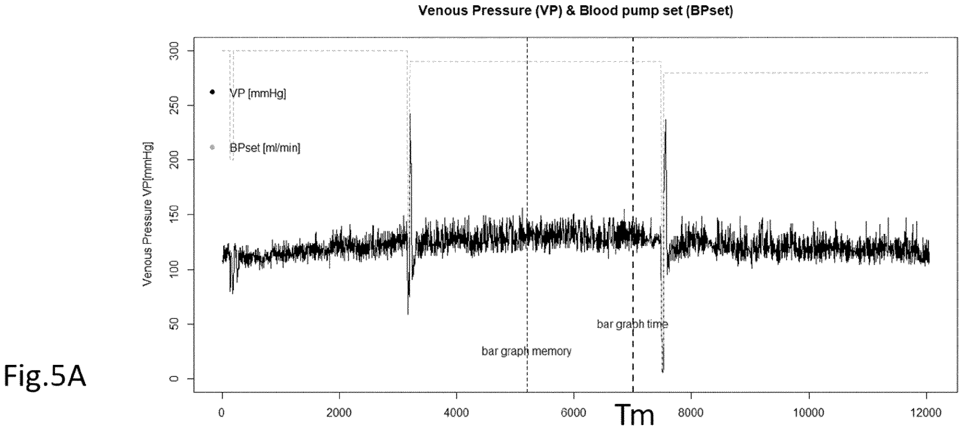

Figure 5A shows a more realistic depiction of the measurement graph of a measurement outcome for venous pressure over time. The rightmost dashed vertical line again indicates the time at which the view of the display 1 shown in figure 5B is shown. It thus forms the measurement time for figure 5B.

Figure 5B depicts the state of the display 1 resulting from the graph shown in figure 5A at time tm. Here the pressure is subject to a much larger fluctuation. Thus, more display regions are colorized. Here display region 3 corresponds to the first display region.

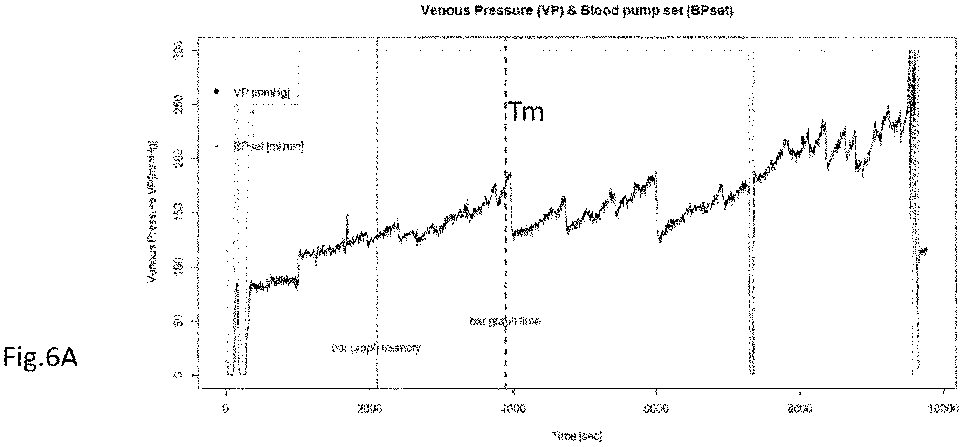

Figure 6A shows a more realistic depiction of the measurement graph of a measurement outcome for venous pressure over time, wherein there is a clear upward trend in the measurement signals is exhibited globally.

Figure 6B depicts the state of the display 1 resulting from the graph shown in figure 6A at time tm. Here, the clear global upward trend is reflected in the fact that the a large number of display regions exhibit a change in color intensity from the initial value of 0. Further, the degree of local fluctuations is reflected in the small gradient of color intensity across the colorized display regions. Here display region 3 corresponds to the first display region.

List of reference signs:

| | |
|---|---|
| 1 | display; |
| 2, 3, 4, 5 | display-regions; |
| 6, 7 | range of measurement parameter; |
| 8 | region of measurement parameter; |
| Tm | Measurement time |
| 9 | Alarm threshold |

**Claims**

1.    Method for processing, preferably periodical, measurement signals, in particular from a measurement device for blood pressure, to be displayed on a user device comprising at least one visual user interface, the method comprising the following steps:

8

a) receiving a measurement signal from at least one sensor at a first time;

b) associating the measurement signal with a first display-region of a plurality of display-regions of a display;

c) calculating, based

- on the measurement signal,
- on the first time and
- on a previous display-state-parameter of at least one of the plurality of display regions at a second time,

at least one display-state-parameter of the at least one of the plurality of display-regions,

d) assigning a new display-state-parameter to at least one of the plurality of display-regions, based on the display-state-parameter calculated according to c).

2. Method according to claim 1, wherein each of the display regions is indicative of a range of at least one measurement parameter.

3. Method according to one of the previous claims, wherein the method comprises an initialization step of predefining a plurality of display regions, in particular based on a region of at least one measurement parameter, wherein the plurality of display regions preferably covers the entire range of the at least one measurement parameter.

4. Method according to one of the previous claims, wherein the calculation of the at least one display-state-parameter in step c) comprises adding a predetermined contribution-parameter to the at least one display-state-parameter, when the measurement signal was found to correspond to the associated display-region.

5. Method according to one of the previous claims, wherein the calculation of the at least one display-state-parameter in step c) comprises the application of a damping-factor to the at least one display-state-parameter, wherein the damping factor depends on a retaining time and the first and/or second time, wherein the damping factor preferably does not depend on a recorded measurement signal.

6. Method according to one of the previous claims, in particular claims 4 and 5, wherein the predetermined contribution-parameter and/or damping factor is chosen such that the display-state-parameter is bounded from above and/or below.

7. Method according to one of the previous claims, in particular claims 5 and 6, wherein the damping factor is chosen, such that display-state-parameters tend to decrease over time, wherein the decrease can preferably be adjusted to depend on the retaining time according to one of the following: constant, linear, polynomial, exponential, logarithmic.

8. Method according to one of the previous claims, comprising a further step of automatically choosing a predetermined display-state-parameter for the first display-region.

9. Method according to one of the previous claims, further comprising a step of calibrating the user device such that every pair of distinct display-state-parameters leads to a visually distinguishable pair of display-states.

10. Method according to one of the previous claims, in particular claim 2, wherein the plurality of display-regions correspond to intervals of a blood pressure.

11. Method according to one of the previous claims, wherein a plurality of display-state-parameters are defined, in particular stored, as a state vector, wherein each of the components of the state vector is associated to a display-region and/or a measurement parameter.

12. Method according to one of the previous claims, wherein the display-state-parameters represent at least one of the following: a display-brightness, a display-color, a color intensity.

13. Method according to one of the previous claims, in particular claim 11, wherein the damping factor is applied by the multiplication of every vector component with the damping factor.

14. System comprising at least one sensor and a user device comprising at least one visual user interface, preferably a measurement device for blood pressures, as well as a processor controlling the visual user interface based on a measurement signal from the sensor, wherein the system is configured to carry out a method according to one of the claims 1 to 13.

**15.** Haemodialysis or blood circuit machine comprising a system according to claim 14.

**16.** Computer readable medium storing instructions that when executed by at least one processor cause the at least one processor to implement a method according to claims 1 to 13.

Fig.1

Fig. 2

Fig.3

EP 4 618 094 A1

Fig.4A

Venous Pressure (VP) & Blood pump set (BPset)

Fig.4B

Fig.5A

Fig.5B

Fig.6A

Fig.6B

Europäisches
Patentamt

European
Patent Office

Office européen
des brevets

# EUROPEAN SEARCH REPORT

Application Number

EP 24 16 3216

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/385434 A1 (YUDS DAVID [US] ET AL) 19 December 2019 (2019-12-19) * paragraphs [0037] - [0040]; figure 1 * ----- | 1-16 | INV. G16H20/40 |

TECHNICAL FIELDS
SEARCHED      (IPC)

G16H

The present search report has been drawn up for all claims

| Place of search | Date of completion of the search | Examiner |
|---|---|---|
| Munich | 7 August 2024 | Reinbold, Bernhard |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

# EP 4 618 094 A1

## ANNEX TO THE EUROPEAN SEARCH REPORT
## ON EUROPEAN PATENT APPLICATION NO.

EP 24 16 3216

This annex lists the patent family members relating to the patent documents cited in the above-mentioned European search report.
The members are as contained in the European Patent Office EDP file on
The European Patent Office is in no way liable for these particulars which are merely given for the purpose of information.

07-08-2024

| Patent document cited in search report | Publication date | Patent family member(s) | | Publication date |
|---|---|---|---|---|
| US 2019385434 A1 | 19-12-2019 | CA | 3099442 A1 | 26-12-2019 |
| | | CN | 112312937 A | 02-02-2021 |
| | | EP | 3773785 A1 | 17-02-2021 |
| | | US | 2019385434 A1 | 19-12-2019 |
| | | WO | 2019245918 A1 | 26-12-2019 |

For more details about this annex : see Official Journal of the European Patent Office, No. 12/82